# FASCICULE DE BREVET EUROPEEN

(11) **EP 0 541 587 B1**
(45) Date de publication et mention de la délivrance du brevet: **28.12.1994**
(21) Numéro de dépôt: 91912913.0
(22) Date de dépôt: 26.07.1991
(51) Int. Cl.: G01L 9/00

(54) **CAPTEUR DE PRESSION A FIBRE OPTIQUE**
DRUCKWANDLER MIT OPTISCHER FASER
FIBRE-OPTICAL PRESSURE SENSOR

(30) Priorité: 27.07.1990 BE 9000752
(43) Date de publication de la demande: 19.05.1993
(73) Titulaire: CHEVALIER PHOTONICS, B-1070 Bruxelles (BE)
(72) Inventeur: CHEVALIER, Philippe, B-9731 Nazareth (BE)
(74) Mandataire: Van Malderen, Michel
(86) Numéro de dépôt international: BE9100052
(87) Numéro de publication internationale: WO9202796

(56) Documents cités:
- EP-A- 0 178 806
- GB-A- 2 234 344
- US-A- 4 691 709
- Patent Abstracts of Japan, vol. 12, no. 188 (P-711)(3035), 2 June 1988 & JP-A-62294928

## Description

### Objet de l'invention

La présente invention se rapporte à un capteur de pression utilisant des fibres optiques pour la transmission par faisceaux lumineux et s'étend aux dispositifs de détection ou de mesure munis d'un tel capteur.

### Description de l'art antérieur

Les capteurs de pression peuvent être classés en deux catégories, à savoir les capteurs extrinsèques et intrinsèques.

Dans les capteurs extrinsèques, la fibre optique est passive et n'est employée que pour transmettre la lumière d'un point à un autre. Dans les capteurs intrinsèques, la fibre optique est active et est employée pour quantifier la grandeur physique (pression, température, pH,...).

Dans le brevet US-A-4 691 709 (Cohen), on décrit un capteur pouvant mesurer la vitesse du sang et la pression statique. Ce capteur est basé sur des pertes d'énergie lumineuse résultant du contact d'une mousse élastique avec la fibre optique. Les pertes augmentent en fonction de l'élargissement de la zone de contact.

Ce mode de fonctionnement est à qualifier intrinsèque puisque la caractéristique de base de la fibre optique, la réflexion totale est altérée au contact de la mousse.

Ce capteur fait aussi appel au mode de fonctionnement extrinsèque puisqu'il emploie une membrane réfléchissante disposée à l'extrémité de la fibre optique et dont le rayon de courbure varie en fonction de la pression.

Cependant, si l'on désire produire ce capteur en grande série, le positionnement de cette membrane dans le capteur occasionne des problèmes technologiques dont l'impact sur le coût n'est pas négligeable.

La demande de brevet JP-A-62 294 928 (Idemitsu Kosan Co. LTD) décrit un atténuateur optique mesurant la pression sur base de la variation de la transmission du système.

L'atténuateur est constitué d'une dispersion de colorant dans un élastomère. La distribution spatiale des particules de colorants est modifiée par la pression exercée sur l'élastomère de telle façon qu'une variation de la transmission du système en résulte.

Ce capteur extrinsèque se base en majeure partie sur une fonction de transfert définie par la loi de Behr-Lambert, ce qui revient à dire que cette fonction est du type exp(-ax) où a est le coefficient d'atténuation et x est le chemin optique parcouru par le faisceau lumineux.

Cependant, dans une production en série, un tel capteur ne peut garantir une distribution reproductible du colorant dans l'élastomère et par conséquent on ne peut obtenir une configuration reproductible du capteur.

De plus, un tel capteur de transmission ne peut fournir un rapport signal/bruit. suffisamment élevé et est difficilement transposable à une mesure de pression artérielle à cause de l'encombrement nécessaire.

On connaît également des capteurs de pression faisant appel à des fibres optiques et dont le fonctionnement général est basé sur le déplacement de miroirs fixés sur des membranes ou sur des pistons soumis aux forces de pression [Scheggi (Institut di Ricerca Sulle Onde Electromagnetische CNR-Firenze, Italy) et Matsumoto (J. Med. Eng. and Tech. 2, 239 (5) Sept. 1978)].

Le déplacement des miroirs modifie la distribution de l'intensité de lumière dans la fibre optique et permet ainsi de mesurer la pression exercée sur la membrane ou le piston.

La demande de brevet EP-A-0178806 (Sperry Corporation) décrit un capteur de type intrinsèque utilisant un coupleur 2 x 2 où le rapport d'énergie des canaux de sortie est influencé par la variation de l'indice de réfraction en fonction de la pression.

Des capteurs de pression employant des fibres bifurquées où une extrémité est connectée à une membrane ont été décrits par Hansen (CLEO'81 Tech. Dig. FI4, 176 (1981).

Au lieu de se baser sur le déplacement d'une membrane, Lawson (Opt. Lett. 8, 286 (5) 1983) et la demande de brevet JP-A-56 7034 (Tokyo Shibaura Denki K.K.) décrivent un capteur utilisant le rayon de courbure de la membrane réfléchissante. Le rayon de courbure de la membrane varie dans ce cas en fonction de la pression.

Le brevet US-A-3 273 447 (Frank) décrit une fibre optique terminée par un bulbe en une matière élastique à la déformation et pouvant être recouverte d'une couche réfléchissante.

Ce bulbe a une forme asphérique et a subi une prétension en fonction de la pression à mesurer et en fonction des caractéristiques élastiques de la matière. En fonction de la pression exercée, le bulbe se déforme et réfléchit plus ou moins la lumière dans la fibre optique.

Selon une variante d'exécution, une cavité peut être introduite dans ce bulbe. La cavité reçoit une forme asphérique de telle façon qu'un faisceau de lumière suit un chemin autour de la cavité et est renvoyé dans la fibre optique.

Toute pression exercée sur le bulbe a comme effet d'accroître les pertes optiques du fait que le faisceau ne sera plus réfléchi totalement en certains endroits de la surface, puisqu'à ces endroits le rayon de courbure aura changé.

Cependant, un tel capteur présente les désavantages d'offrir une sensibilité insuffisante et d'avoir une configuration non reproductible lorsqu'il est produit en série.

### Buts de l'invention

La présente invention vise à fournir un capteur d'un prix de revient très faible en comparaison avec ceux proposés actuellement.

Un autre but de la présente invention vise à fournir des capteurs de pression qui conviennent à des applications médicales, qui satisfont donc à des critères de faible dimensionnement, qui peuvent être implantés, et qui sont donc tolérés par l'organisme.

Un autre but de la présente invention est de fournir un capteur de pression d'une précision et d'une fiabilité suffisantes pour une application médicale.

Un but complémentaire de la présente invention est de fournir un capteur de pression presentant une configuration reproductible lors d'une production en série.

### Eléments caractéristiques de l'invention

L'invention concerne essentiellement un capteur de pression connectable à une fibre optique permettant le transfert d'un faisceau lumineux et qui est constitué d'une matière essentiellement élastique à la deformation et transparente optiquement aux longueurs d'ondes utilisées, de préférence un élastomère, comportant au moins une inclusion constituée par au moins un tronçon de fibre optique et dont la fonction de transfert est modifiée sous l'effet de la déformation.

Avantageusement, l'inclusion est de forme toroïdale, de préférence de forme cylindrique et présente un diamètre compris entre 10 µm a 500 µm.

Cette inclusion peut être constituée de verre organique, inorganique, d'un fluide et/ou d'un élastomère différent de l'élastomère entourant ladite inclusion.

Avantageusement, le tronçon de fibre optique présente un axe de rotation formant un angle avec l'axe de rotation de la dite fibre optique permettant le transfert des faisceaux lumineux. Cet angle est de préférence de l'ordre de 90°.

### Brève description des figures

- La figure 1 représente une vue schématique du système de détection classique comprenant un capteur de pression.
- La figure 2 représente en détail le module de détection.
- La figure 3 représente une vue en coupe longitudinale du capteur de pression selon la présente invention.
- La figure 4a représente une vue en coupe d'une forme d'exécution préférée du capteur selon l'invention.
- La figure 4b représente une vue en plan d'une forme d'exécution préférée du capteur selon l'invention, correspondant à la figure 4a.

### Description d'un mode préféré de l'invention

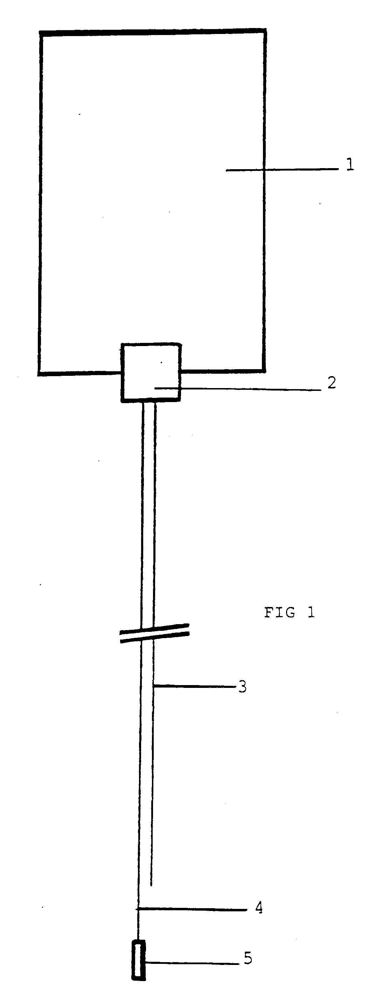

La figure 1 représente une vue schématique d'un système de détection classique.

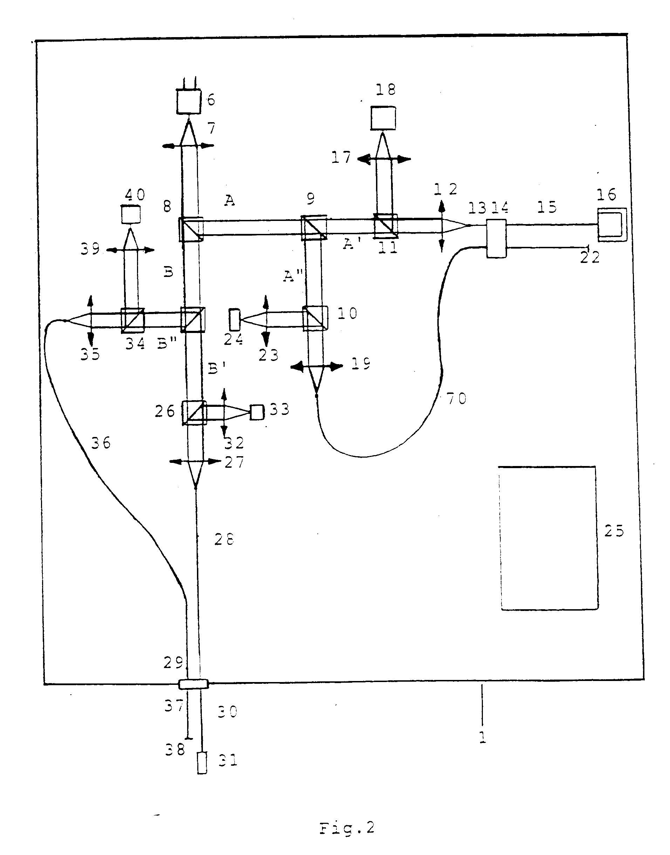

Le repère numerique 1 représente le module de détection qui est représenté plus en detail à la figure 2.

Afin d'obtenir une mesure fiable, il est classiquement nécessaire d'effectuer constamment une compensation pour éliminer les signaux venant d'une modification des paramètres intrinsèques de la fibre de transmission et d'autres perturbations du signal.

A cet effet, une fibre 3 dite de compensation est prévue et est disposée en parallèle avec la fibre 4 de transmission au bout de laquelle est placée le capteur de pression 5.

Au bout de la fibre 3 de compensation est disposé un miroir qui permet une réflexion totale du rayon lumineux.

Un connecteur 2 de fibre optique permet de raccorder et d'enlever les deux fibres optiques 3 et 4.

Selon un premier mode d'exécution, on préfère généralement travailler en réflexion. Mais il est également possible d'envisager un fonctionnement du capteur de pression par fibre optique en transmission du faisceau lumineux.

Toute pression exercée sur le capteur se traduit par une modification de l'intensité lumineuse réfléchie et/ou diffusée dans la fibre optique de transmission.

Le faisceau lumineux provient d'une source émettrice lumineuse 6 (laser/LED). Le faisceau initial est collimé par la lentille 7 et puis séparé en deux faisceaux A et B par un séparateur 8.

Le faisceau A est séparé par un séparateur 9 en A′ et A˝.

Le faisceau A′ traverse un séparateur 11 et est focalisé par une lentille 12 sur une fibre optique 13. Cette fibre optique est connectée au connecteur 14. Une fibre optique 15 conduit le faisceau lumineux à un capteur de pression 16. Ce capteur de pression est placé sous pression calibrée. Le signal venant de ce capteur est renvoyé dans la fibre optique 15 et transmis par le connecteur 14 à la fibre optique 13. Le faisceau est ensuite collecté par la lentille 12 et transmis par le séparateur 11 à la lentille 17. Le faisceau est ensuite focalisé sur la photodiode 18.

Le faisceau A˝ est transmis au séparateur 10 et est ensuite focalisé par la lentille 19 dans la fibre optique 20. La fibre optique 20 est reliée au connecteur 14 et le faisceau est transmis à la fibre optique 21. Cette fibre optique 21 est parallèle à la fibre optique 15 et la fibre optique 21 est terminée par un miroir 22.

Le faisceau est réfléchi par le miroir 22 et transmis par la fibre 21 au travers du connecteur 14 dans la fibre optique 20. La lentille 19 capte le faisceau et le transmet par le séparateur 10 à la lentille 23. La lentille 23 focalise le faisceau sur la photodiode 24.

Les signaux venant des photodiodes 18 et 24 sont traités par un processeur 25. L'opération principale effectuée par le processeur est de faire le rapport des deux signaux.

La calibration interne se base sur l'enregistrement du signal en fonction de la pression exercée sur le capteur 16. Cette fonction est mémorisée.

Une pression de travail est choisie et celle-ci sera la base pour determiner la difference entre le signal généré par le capteur 16 et le capteur 31. Lors de la calibration, les capteurs 31 et 16 sont placés sous la même pression.

Le faisceau B est séparé par un séparateur 10 en B′ et B˝. Le faisceau B′ traverse un séparateur 26 et est focalisé par une lentille 27 sur une fibre optique 28. Cette fibre optique est connectée au connecteur 29. Une fibre optique 30 conduit le faisceau lumineux à un capteur de pression 31. Un tel capteur de pression peut être implanté sur un patient. Le signal venant de ce capteur est renvoye dans la fibre optique 30 et transmise par le connecteur 29 à la fibre optique 28. Le faisceau est ensuite collecté par la lentille 27 et transmis par le séparateur 26 à la lentille 32. Le faisceau est ensuite focalisé sur la photodiode 33.

Le faisceau B˝ est transmis au séparateur 34 et est ensuite focalisé par la lentille 35 dans la fibre optique 36. La fibre optique 36 est reliée au connecteur 29 et le faisceau est transmis à la fibre optique 37. Cette fibre optique 37 est parallele à la fibre optique 30 et la fibre optique 37 est terminee par un miroir 38.

Le faisceau est réfléchi par le miroir 38 et transmis par la fibre 37 au travers du connecteur 29 dans la fibre optique 36. La lentille 35 capte le faisceau et le transmet par le séparateur 34 à la lentille 39. La lentille 39 focalise le faisceau sur la photodiode 40.

Les signaux venant des photodiodes 33 et 40 sont traités par un processeur 25. L'opération principale effectuée par le processeur est de faire le rapport des deux signaux.

Le processeur traitera en finale les signaux venant des photodiodes 18,24, 33 et 40 pour en extraire la valeur de pression.

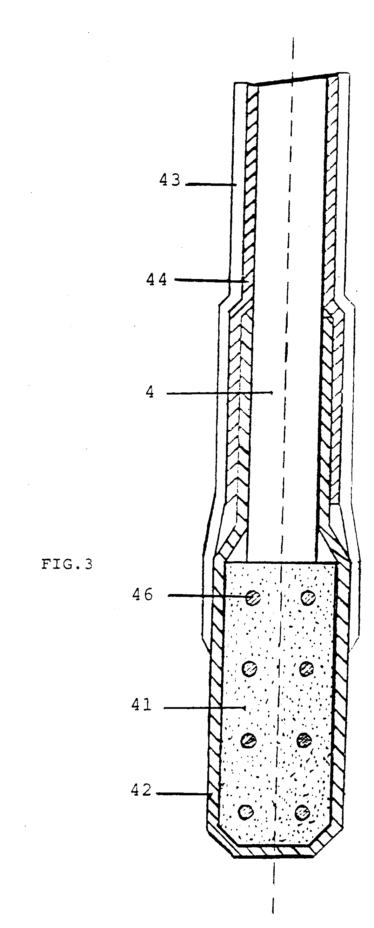

La figure 3 représente une vue en coupe longitudinale d'un capteur de pression selon l'invention qui peut être incorporé dans un dispositif du type décrit ci-dessus.

Un bloc en élastomère 41 et la fibre optique 4 sont collés l'un à l'autre. Une fine membrane 42, par exemple en latex, de protection recouvre tout le capteur. Un manchon 44 est utilisé pour fixer la gaine 42 à la fibre optique 54.

Le capteur est constitué de préférence d'un silicone. Ces capteurs étant plus particulièrement destinés à des applications médicales, par exemple, à la mesure de la pression sanguine ou respiratoire, il est nécessaire que le capteur de pression soit placé dans un catheter 43 toléré par l'organisme humain. En général, ce cathéter est constitué d'une gaine en latex. Il est bien entendu que pour une telle application les dimensions d'un tel capteur doivent également être telles qu'elles puissent être tolérées par l'organisme humain sous forme d'implant, c'est-à-dire de l'ordre du millimètre.

Les pressions détectées par les capteurs selon l'invention sont comprises entre 0 et 300 mm de mercure.

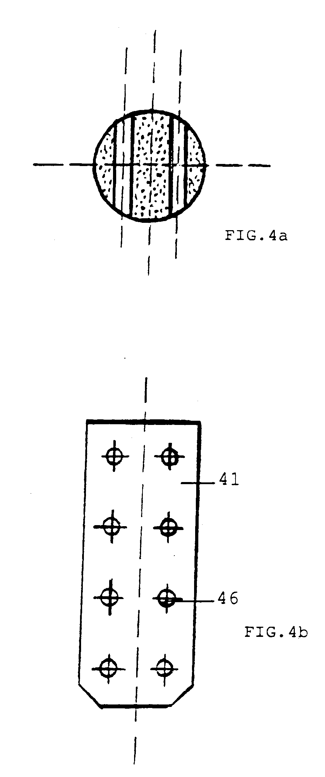

Les figures 4a et 4b représentent une forme d'exécution préférée du capteur selon l'invention.

Le capteur est constitué d'un bloc de silicone 41 dans lequel apparaissent des petits cylindres de fibre optique de silice disposés essentiellement à la perpendiculaire du faisceau lumineux. Le rayon lumineux émis subit des phénomènes de réflexion, de diffusion et de focalisation. Lorsque l'on exerce une pression sur le capteur, la distance entre les inclusions varie et de ce fait, la focale du système optique varie ce qui entraîne une variation de l'énergie lumineuse collectée par la fibre de transmission.

Il est bien entendu que la présente invention ne se limite pas aux exemples d'exécution donnés uniquement à titre d'illustration. En particulier, la présente invention ne se limite pas à des applications médicales telles que décrites précédemment.

On peut également envisager de nombreuses autres applications techniques et industrielles dans des domaines extrêmement variés, telles que la mesure des vibrations des machines, de la densité du trafic, mesure de vitesse lors de competitions sportives, detection de paramètres de sécurité de ponts et d'ouvrages d'art, etc.

## Revendications

1. Capteur de pression (1) connectable à une fibre optique (4) permettant le transfert d'un faisceau lumineux, caractérisé en ce qu'il est constitué d'une matière (41) essentiellement élastique à la deformation et transparente optiquement aux longueurs d'ondes utilisées comportant au moins une inclusion constituée par au moins un tronçon de fibre optique (46) et dont la fonction de transfert est modifiée sous l'effet de la déformation.

2. Capteur selon la revendication 1 caractérisé en ce que la matiere essentiellement élastique est constituée par un élastomère.

3. Capteur selon la revendication 1 ou 2 caractérisé en ce que l'inclusion est de forme toroïdale.

4. Capteur selon la revendication 3 caractérisé en ce que l'inclusion est de forme cylindrique.

5. Capteur selon l'une quelconque des revendications 1 à 4 caractérisé en ce que l'inclusion présente un diamètre compris entre 10 et 500 µm.

6. Capteur selon l'une quelconque des revendications 1 à 5 caractérisé en ce que l'inclusion est constituée de verre organique, inorganique, d'un fluide et/ou d'un élastomère différent de l'élastomère entourant ladite inclusion.

7. Capteur selon l'une quelconque des revendications 1 à 6 caractérisé en ce que le tronçon de fibre optique présente un axe de rotation formant un angle avec l'axe de rotation de ladite fibre optique permettant le transfert des faisceaux lumineux.

8. Capteur selon la revendication 7 caractérisé en ce que ledit angle est de l'ordre de 90°.

9. Dispositif de détection ou de mesure caractérisé en ce qu'il comporte au moins un capteur de pression selon l'une quelconque des revendications 1 à 8.

10. Application du dispositif selon la revendication 9 à la mesure de la pression sanguine ou respiratoire par implantation sur un patient.

## Patentansprüche

1. Druck-Meßwertgeber (1), der an eine Lichtleitfaser (4) anschließbar ist, die die Übertragung eines Lichtbündels ermöglicht, dadurch gekennzeichnet, daß er aus einem im wesentlichen bei Verformung elastischen und bei den verwendeten Wellenlängen optisch transparenten Material (41) besteht, das mindestens einen aus mindestens einem Lichtleitfaser-Abschnitt (46) bestehenden Einschluß umfaßt, dessen Übertragungsfunktion bei Verformung verändert wird.

2. Druck-Meßwertgeber gemäß Anspruch 1, dadurch gekennzeichnet, daß das im wesentlichen elastische Material aus einem Elastomer besteht.

3. Druck-Meßwertgeber gemäß Anspruch 1 oder 2, dadurch gekennzeichnet, daß der Einschluß eine toroide Form hat.

4. Druck-Meßwertgeber gemäß Anspruch 3, dadurch gekennzeichnet, daß der Einschluß eine zylindrische Form hat.

5. Druck-Meßwertgeber gemäß irgendeinem der Ansprüche 1 bis 4, dadurch gekennzeichnet, daß der Einschluß einen Durchmesser zwischen 10 und 500 µm aufweist.

6. Druck-Meßwertgeber gemäß irgendeinem der Ansprüche 1 bis 5, dadurch gekennzeichnet, daß der Einschluß aus organischem oder anorganischem Glas, einem Fluid und/oder einem Elastomer besteht, wobei dieses Elastomer verschieden von dem Elastomer ist, das den Einschluß umgibt.

7. Druck-Meßwertgeber gemäß irgendeinem der Ansprüche 1 bis 6, dadurch gekennzeichnet, daß der LichtleitfaserAbschnitt eine Drehachse aufweist, die mit der Drehachse der Lichtleitfaser, die die Übertragung der Lichtbündel ermöglicht, einen Winkel bildet.

8. Druck-Meßwertgeber gemäß Anspruch 7, dadurch gekennzeichnet, daß der Winkel ungefähr 90° beträgt.

9. Nachweis- oder Meßvorrichtung, dadurch gekennzeichnet, daß sie mindestens einen Druck-Meßwertgeber gemäß irgendeinem der Ansprüche 1 bis 8 umfaßt.

10. Anwendung der Vorrichtung gemäß Anspruch 9 zur Messung des Blutdrucks oder Atmungsdrucks durch Implantation bei einem Patienten.

## Claims

1. Pressure sensor (1) which can be connected to an optical fiber (4) allowing the transfer of a light beam, characterized in that it consists of a material (41) which is essentially elastic to deformation and optically transparent to the wavelengths used comprising at least one inclusion consisting of at least one segment of optical fiber (46) and whose transfer function is modified under the effect of deformation.

2. Sensor according to claim 1, characterized in that the essentially elastic material consists of an elastomer.

3. Sensor according to claim 1 or 2, characterized in that the inclusion is of toroidal shape.

4. Sensor according to claim 3 characterized in that the inclusion is of cylindrical shape.

5. Sensor according to any one of claims 1 to 4, characterized in that the inclusion has a diameter between 10 and 500 µm.

6. Sensor according to any one of claims 1 to 5, characterized in that the inclusion consists of organic or inorganic glass, of a fluid and/or of a different elastomer from the elastomer surrounding the said inclusion.

7. Sensor according to any one of claims 1 to 6 characterized in that the segment of optical fiber has an axis of rotation forming an angle with the axis of rotation of the said optical fiber allowing the transfer of light beams.

8. Sensor according to claim 7, characterized in that the said angle is of the order of 90°.

9. Detection or measurement device characterized in that it comprises at least one pressure sensor according to any one of claims 1 to 8.

10. Application of the device according to claim 9 to measuring the blood or respiratory pressure by implantation on a patient.
